(19) Europäisches Patentamt / European Patent Office / Office européen des brevets

(11) **EP 3 435 858 B1**

(12) **EUROPEAN PATENT SPECIFICATION**

(45) Date of publication and mention of the grant of the patent:
**08.07.2020 Bulletin 2020/28**

(21) Application number: **16712341.3**

(22) Date of filing: **30.03.2016**

(51) Int Cl.:
$A61B\ 5/053^{(2006.01)}$  $A61B\ 5/0452^{(2006.01)}$
$A61B\ 5/042^{(2006.01)}$  $A61B\ 5/0215^{(2006.01)}$
$A61B\ 5/029^{(2006.01)}$  $A61N\ 1/05^{(2006.01)}$
$A61B\ 5/00^{(2006.01)}$  $A61B\ 5/02^{(2006.01)}$
$A61B\ 5/107^{(2006.01)}$  $A61B\ 5/046^{(2006.01)}$
$A61B\ 5/0464^{(2006.01)}$  $A61M\ 25/00^{(2006.01)}$
$A61B\ 5/06^{(2006.01)}$

(86) International application number:
**PCT/EP2016/056933**

(87) International publication number:
**WO 2017/167360 (05.10.2017 Gazette 2017/40)**

(54) **DEVICES TO ASSESS INFARCTED MYOCARDIAL TISSUE BY MEASURING ELECTRICAL IMPEDANCE DURING THE CARDIAC CYCLE**

GERÄTE ZUR BEURTEILUNG VON MYOKARDINFARKTGEWEBE DURCH MESSUNG DER ELEKTRISCHEN IMPEDANZ WÄHREND DES HERZZYKLUS

DISPOSITIFS D'ÉVALUATION D'UN TISSU MYOCARDIQUE INFARCI PAR MESURE DE L'IMPÉDANCE ÉLECTRIQUE DURANT LE CYCLE CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.02.2019 Bulletin 2019/06**

(73) Proprietors:
• **Universitat Politècnica de Catalunya**
  **08034 Barcelona (ES)**
• **Fundacio Institut de Recerca de l'Hospital de la Santa Creu i sant Pau**
  **08025 Barcelona (ES)**

(72) Inventors:
• **ROSELL FERRER, Francesc Xavier**
  **08034 Barcelona (ES)**
• **CINCA CUSCULLOLA, Juan**
  **08025 Barcelona (ES)**
• **BRAGÓS BARDIA, Ramón**
  **08034 Barcelona (ES)**
• **AMORÓS FIGUERAS, Gerard**
  **08025 Barcelona (ES)**
• **JORGE VIZUETE, Esther**
  **08025 Barcelona (ES)**

• **GARCÍA SÁNCHEZ, Tomàs**
  **E-08018 Barcelona (ES)**
• **SÁNCHEZ TERRONES, Benjamín**
  **Boston, MA 02115 (US)**

(74) Representative: **ZBM Patents - Zea, Barlocci & Markvardsen**
  **Rambla Catalunya, 123**
  **08008 Barcelona (ES)**

(56) References cited:
WO-A1-2008/105691  WO-A1-2014/008489
US-A1- 2004 260 278  US-A1- 2006 184 060
US-A1- 2007 191 901  US-A1- 2010 204 585
US-A1- 2011 144 510

• ESTHER JORGE ED - MITAL R ET AL: "Early detection of acute transmural myocardial ischemia by the phasic systolic-diastolic changes of local tissue electrical impedance", AMERICAN JOURNAL OF PHYSIOLOGY, AMERICAN PHYSIOLOGICAL SOCIETY, USA, vol. 310, no. 3, 25 November 2015 (2015-11-25), pages H436-H443, XP008182498, ISSN: 1522-1539, DOI: 10.1152/AJPHEART.00754.2015

- POLLARD ANDREW E ET AL: "A New Approach for Resolution of Complex Tissue Impedance Spectra in Hearts", IEEE TRANSACTIONS ON BIOMEDICAL ENGINEERING, IEEE SERVICE CENTER, PISCATAWAY, NJ, USA, vol. 60, no. 9, 1 September 2013 (2013-09-01), pages 2494-2503, XP011524033, ISSN: 0018-9294, DOI: 10.1109/TBME.2013.2258917 [retrieved on 2013-08-16]
- Y. SALAZAR ET AL: "Transmural Versus Nontransmural In Situ Electrical Impedance Spectrum for Healthy, Ischemic, and Healed Myocardium", IEEE TRANSACTIONS ON BIOMEDICAL ENGINEERING., vol. 51, no. 8, 1 August 2004 (2004-08-01) , pages 1421-1427, XP055324987, PISCATAWAY, NJ, USA. ISSN: 0018-9294, DOI: 10.1109/TBME.2004.828030
- SANCHEZ B ET AL: "Novel Estimation of the Electrical Bioimpedance Using the Local Polynomial Method. Application to In Vivo Real-Time Myocardium Tissue Impedance Characterization During the Cardiac Cycle", IEEE TRANSACTIONS ON BIOMEDICAL ENGINEERING, IEEE SERVICE CENTER, PISCATAWAY, NJ, USA, vol. 58, no. 12, 1 December 2011 (2011-12-01), pages 3376-3385, XP011408580, ISSN: 0018-9294, DOI: 10.1109/TBME.2011.2166116
- BRAGOS R ET AL: "Characterisation of dynamic biologic systems using multisine based impedance spectroscopy", IMTC 2001. PROCEEDINGS OF THE 18TH. IEEE INSTRUMENTATION AND MEASUREMENT TECHNOLOGY CONFERENCE. BUDAPEST, HUNGARY, MAY 21 - 23, 2001; [IEEE INSTRUMENTATION AND MEASUREMENT TECHNOLOGY CONFERENCE. (IMTC):], NEW YORK, NY : IEEE, US, vol. 1, 21 May 2001 (2001-05-21), pages 44-47, XP010546659, DOI: 10.1109/IMTC.2001.928785 ISBN: 978-0-7803-6646-6

## Description

The present disclosure relates to devices to assess infarcted tissue by measuring electrical impedance. More specifically, the devices may be used to recognize the extent and deepness of an infarcted tissue, such as for example myocardial infarcted tissue.

## BACKGROUND ART

Ventricular arrhythmias are responsible of approximately 60% of sudden deaths in patients with a previously cardiac infarction. Radiofrequency ablation of the arrhythmogenic foci is able to treat around 50-80% of the patients with postinfarction ventricular arrhythmias. The success rate of this procedure could be increased by improvements in the identification and localization of the arrhythmogenic foci in the clinical practice. The clinical intracardiac navigation systems used nowadays (for example: (i) CARTO®, provided by Biosence Webster®, (ii) NavX™, provided by St. Jude Medical™ , or (iii) Rhythmia™, provided by Boston Scientific™) locate the postinfarction scar by local measures of voltage using intracavitary electrocatheters. A major drawback of the voltage measurements is that they cannot determine if the scar is completely transmural or not. Another drawback is that these voltage measurements depend on the wave front activation pattern, that can change if the patient suffers and ectopic arrhythmic episode.

The characterization of biological substrates by means of electrical impedance provides relevant physiological information about the pathological status of the tissues. It has been reported that normal and infarcted myocardium can be recognized by measuring the myocardial electrical impedance (module and phase angle) using an intracardiac electrocatheter, see e.g. "Warren M, et al. Percutaneous electrocatheter technique for on-line detection of healed transmural myocardial infarction. Pacing Clin Electrophysiol. 2000;23:1283-1287" *(Warren 2000')*. As compared with the normal myocardium, the necrotic infarct scar shows a lower impedance module and a flat phase angle deviation. Measuring the electrical impedance as an indicator of the structural condition of the cardiac tissue has been already proposed in the past. Previous documents (US5494042A; US2001018608A; US5485849) have described systems and methods using the impedance to identify infarcted regions of heart. However, these systems measure the impedance at a single frequency or at few selected frequencies (between 5 Hz and 50 kHz). With these "old" techniques, only few impedance measurements could be taken during the cardiac cycle due to the time required to inject the wide current spectrum by frequency sweeping. The cardiac movement during contraction and relaxation induces impedance changes that increase the dispersion of impedance measures and this curtails the capacity of the system to recognize the structural derangement.

It is also noted that prompt coronary artery reperfusion in patients with acute myocardial infarction favors cell survival and ultimately promotes the development of heterogeneous transmural infarct scar (see Francone M., et al. Impact of primary coronary angioplasty delay on myocardial salvage, infarct size, and microvascular damage in patients with ST-segment elevation myocardial infarction. Insight from cardiovascular magnetic resonance. J. Am. Coll. Cardiol. 2009;54:2145-2153). The interspaced islands of surviving myocytes may act as slow conducting pathways thereby favoring re-entrant arrhythmias (Arenal A et al. Tachycardia-related channel in the scar tissue in patients with sustained monomorphic ventricular tachycardias: Influence of the voltage scar definition. Circulation 2004;110:2568-2574.; Nakahara S., et al. Characterization of the arrhythmogenic substrate in ischemic and nonischemic cardiomyopathy. Implications for catheter ablation of hemodynamically unstable ventricular tachycardia. J. Am. Coll. Cardiol. Elsevier Inc.; 2010;55:2355-2365) and increasing mortality (Yan A.T., et al. Characterization of the peri-infarct zone by contrast-enhanced cardiac magnetic resonance imaging is a powerful predictor of post-myocardial infarction mortality. Circulation 2006;114:32-39). Postinfarction ventricular arrhythmias can be suppressed by electrical catheter ablation of the arrhythmogenic substrate but this procedure requires an accurate delineation of the infarct scar and a precise location of the target ablation sites scattered within the infarcted region. The cardiac navigation systems employed in the catheter ablation procedures utilize the mapping of low voltage endocardial electrograms to delineate the borders of the infarct scar although this technique does not allow appropriate discrimination among sites with different degrees of transmural involvement (Codreanu A., et al. Electroanatomic characterization of post-infarct scars. Comparison with 3-Dimensional myocardial scar reconstruction based on magnetic resonance imaging. J. Am. Coll. Cardiol. 2008;52:839-842).

In clinical practice, the heterogeneous nature of the infarct scar may only be assessed accurately by cardiac magnetic resonance imaging (Klein C., et al. Assessment of myocardial viability with contrast-enhanced magnetic resonance imaging: comparison with positron emission tomography. 2002;105:162-167), but previous studies have reported differential biophysical electrical characteristics between the normal myocardium and the infarcted tissue (Warren 2000). Myocardial electrical impedance is a biophysical property of the heart that is influenced by the intrinsic structural characteristics of the myocardial tissue (Sperelakis N., et al. Electrical impedance of cardiac muscle. Circ. Res. 1961;9:1280-1283) as denoted by experimental models of acute and chronic myocardial infarction (Cinca J., et al. Passive transmission of ischemic ST segment changes in low electrical resistance myocardial infarct scar in the pig. Cardiovasc. Res. 1998;40:103-112 ; Fallert MA et al. Myocardial electrical impedance mapping of ischemic sheep hearts and healing aneurysms. Circulation 1993;87:199-207; Salazar Y et al. Transmural versus nontransmural in situ electrical impedance spectrum for healthy, ischemic, and healed myocardium. IEEE Trans. Biomed. Eng. 2004;51:1421-1427). In "Sanchez B et al. A new measuring and identification approach for time-varying bioimpedance using multisine electrical impedance spectroscopy. Physiol. Meas. 2013;34:339-57" a refinement of the impedance measurement technique was demonstrated by applying fast broadband electrical impedance spectroscopy (EIS) that permitted to characterize the changes in myocardial impedance during the cardiac cycle in normal and acute ischemic conditions in the in situ porcine heart (Jorge E., et al. Early detection of acute transmural myocardial ischemia by the phasic systolic- diastolic changes of local tissue electrical impedance. Am. J. Physiol. Heart Circ. Physiol. 2015;310:H436-H443).

## SUMMARY OF THE INVENTION

The aim of the present invention, which is solely defined by the appened claims, is to provide a measuring device for medical applications, which can be used to characterize tissues, for example myocardial tissue structure integrity and solve at least partly the drawbacks and limitations of known systems used in clinical practice. This is achieved by measuring the changes in impedance during the entire cardiac cycle by injecting electrical current with a broadband spectrum.

A further capability of the present invention is to analyze the changes in myocardial impedance during the cardiac cycle in an infarct scar to detect heterogeneous transmural involvement in the infarcted region.

The present invention takes benefit of the measurements of electrical resistivity of heart tissue using the novel technique of fast broadband electrical impedance spectroscopy (EIS) (see "Sanchez B., et al. A new measuring and identification approach for time-varying bioimpedance using multisine electrical impedance spectroscopy. Physiol. Meas. 2013;34:339-57"). This new technique enables time-varying bioimpedance measurements within the entire cardiac cycle, at simultaneous multiple frequencies (between 1 kHz - 1 MHz), obtaining up to 1,000 spectrum measures/sec. With this new procedure it is possible to record the phasic systolic and diastolic changes in myocardial impedance elicited during the cardiac cycle so the movement-induced impedance changes become useful and gives additional information. This increases the accuracy of the technique because it provides more information about the tissue characteristics.

It is proposed to use this new device to recognize the extent and deepness of infarcted tissue by measuring electrical impedance, for example the extent and deepness of chronic myocardial infarcted tissue by measuring the myocardial electrical impedance during the entire cardiac cycle using one or more intracavitary electrocatheters.

The device may be used to assess the extent and deepness of chronic myocardial infarcted tissue by measuring systolic and diastolic myocardial electrical impedance.

In the invention as claimed, a device is disclosed. The device comprises means for selecting an area of interest of a cardiac tissue; means for identifying one or more measurement locations in the selected area of interest; means for placing an electrocatheter probe at the one or more measurement locations; means for providing a broadband signal to the one or more measurement locations using the electrocatheter probe; means for identifying a diastolic phase of the cardiac cycle; means for measuring impedance of the myocardial tissue during the identified diastolic phase; means for identifying a systolic phase of the cardiac cycle; means for measuring impedance of the cardiac tissue during the identified systolic phase; and means for assessing said cardiac tissue based on said diastolic and systolic impedance measurements.

According to an exemplary disclosure, the device comprises an arbitrary waveform generator (AWG) to deliver one or more broadband frequency current signals having an amplitude and a duration lasting over a time period associated with one or more cardiac cycles. The device further comprises a multielectrode probe, coupled to the AWG, configured to apply the broadband frequency current signals in vivo to a cardiac tissue and measure impedance of the cardiac tissue. The device further comprises an acquisition module (AM). The AM comprises an electrocardiograph (ECG) recorder and may comprise a blood pressure recorder. The device further comprises a controller coupled to the AWG and to the AM and configured to receive the impedance measurements during the duration of the broadband signal, to receive the recordings of the acquisition module during the duration of the broadband signal, to identify a systolic or diastolic phase of a cardiac cycle, to correlate the impedance measurements with the identified phase of the cardiac cycle, and to identify the cardiac tissue as transmural or non-transmural in response to said correlation.

Further examples relate to a device and method for mapping the inner (endocardial) regions of the heart, for example an atrial region or a ventricular region, to delineate the extent of necrotic scar. This may be done by measuring the electrical impedance during the entire cardiac cycle after injecting current pulses at multiple frequencies simultaneously. That has a clinical application in the catheter ablation treatment of ventricular arrhythmias in patients with myocardial infarction.

Some advantages of the equipment according to non-claimed examples may be:

- Unlike other impedance mapping techniques, the present invention is based on simultaneous impedance measurements performed at multiple frequencies, e.g. using electrical impedance spectroscopy (EIS), providing more information about the structural condition of the myocardium tissue. Additionally, these simultaneous multifrequency measurements are performed in a relatively short time compared to the cardiac cycle (in the range of 1 ms, for example between 0.1 and 10 ms, or for example between 0.5 and 2 ms) thus allowing to acquire the whole time-frequency information into the cardiac cycle. In this way, the impedance spectrum measurements are performed at known moments of the cardiac cycle eliminating the influence of the myocardium movement. This new device and method allow a more accurate recognition of the extent and transmurality of the infarct scar and permit a better identification of the target sites for electrical ablation of ventricular arrhythmias.

- Unlike the voltage mapping, the impedance mapping is not influenced by the direction of the activation wave front and because of that, it does not require reassessment of the map data whenever an ectopic rhythm supervened during the clinical procedure.

- Unlike the voltage mapping, the impedance mapping can detect the subendocardial, subepicardial and midmyocardial degree of fibrosis. Therefore the impedance mapping can detect if the fibrosis is transmural or non-transmural.

## BRIEF DESCRIPTION OF THE DRAWINGS

Particular disclosures will be described in the following by way of non-limiting examples, with reference to the appended drawings, in which:

FIG. 1 is a schematic representation of the system used to record simultaneously the phasic changes of myocardial electrical impedance, left ventricular (LV) pressure and surface ECG;
FIG. 1B schematically illustrates various example electrocatheter configurations;
FIG. 2 is a representation of the (non-)periodic broadband EIS for the nonparametric-in-time measurement of the time-varying bioimpedance;
FIG. 3 displays an example of a multisine signal and its spectrum;
FIG. 4A represents a resistivity spectrum which has a modulation due to myocardium movement;
FIG. 4B represents the modulation of the resistivity waveform due to myocardium movement;
FIG. 5 is a Wessel plane showing a set of arcs corresponding to several cardiac cycles;
FIG. 6A represents the time course of R0, R∞ obtained by acquiring 60 spectra per second and fitting them to the Cole model for impedance;
FIG. 6B represents the time course of $\alpha$ obtained by acquiring 60 spectra per second and fitting them to the Cole model for impedance;
FIG. 6C represents the time course of fc obtained by acquiring 60 spectra per second and fitting them to the Cole model for impedance;
FIG. 7 is a flow diagram of a method of assessing a cardiac tissue;
**FIG.7A** displays impedance spectra of three regions with three tissue states and their modulation range due to myocardium movement in the three tissue states;
FIG. 7B shows the arcs in the Wessel plane for the three regions.
**FIG.** 8 shows the time-domain of impedance magnitude at a selected frequency (1 kHz) of 4 different tissues states;
FIG. 9 shows magnitude of the normal (NZ), border (BZ) and healed-scar (infarcted, IZ) zones impedance time signal at three different frequencies (1 kHz, 307 kHz and 939 kHz);
**FIG.10** shows the corresponding phase angle at the same frequencies as the ones indicated in FIG. 9;
**FIG.11** represents the resistivity time course at a frequency of 1 kHz, for two tissue state (normal and acute ischemic) together with the synchronously acquired ventricle pressure, its derivative and the ECG;
**FIG.12A** represents the resistivity at a given frequency (top) and the left ventricular pressure (LVP) (bottom);
**FIG. 12B** shows the cycle described by the evolution of the signal in the Resistivity-LVP plane;
**FIG. 13A** shows the Resistivity-LVP loop at baseline and after 30 min. of left anterior descending (LAD) coronary artery occlusion;
**FIG. 13B** shows the Resistivity-LVP loop area mean at different frequencies at baseline and after 30 minutes of ischemia;
FIG. 14 shows the Resistivity-LVP loop for four different tissue-states;
FIG. 15 illustrates a schematic representation of a heart with two electrocatheters;
**FIG. 16A** illustrates the mean value of resistivity (top) and phase angle (bottom) vs. the excitation frequency for three different tissues: healthy tissue (N), non-transmural and transmural infarcted scar (ISN and IST, respectively);
**FIG. 16B** illustrates the mean values of resistivity (top) and phase angle (bottom) at 4 selected frequencies (1, 41, 307 and 1000 kHz) for the three tissue;
FIG. 17 illustrates a linear correlation between myocardial resistivity (upper-left) / phase angle (lower-left) and the percentage of fibrosis;
FIG.18 illustrates the comparative pattern of the Cole impedance model obtained in normal (N: white), non-transmural scar (ISN: grey) and infarcted transmural scar (IST: black);

**FIG. 19A** illustrates myocardial tissue electrical resistivity and ECG in a normal myocardium;

**FIG. 19B** illustrates myocardial tissue electrical resistivity and ECG in a non-transmural infarct scar;

**FIG. 19C** illustrates myocardial tissue electrical resistivity and ECG in a transmural infarct scar;

FIG. 20 is a multiparametric analysis showing that a combination of resistivity and fc improve the predictive ability to discriminate between both fibrotic tissues.

## DETAILED DESCRIPTION OF EXAMPLES

Non-limiting examples of the present disclosure will be described in the following, with reference to the appended drawings.

Examples of the present disclosure provide a system of monitoring myocardial tissue that comprises:

- At least one contact electrode on the body; and
- At least one electrode placed at the tip of an electrocatheter to be inserted through blood vessels or body openings.

The steps comprised to perform an impedance measurement using this system may be:

1- Generate and apply to the patient an alternating broadband electrical current signal through the electrodes of an intracavitary electrocatheter, between an electrode of an intracavitary electrocatheter and an external skin electrode or between electrodes placed in two different electrocatheters.

2- Measure the voltage signals across a given pair of electrodes of the electrocatheter or / and between an electrode of an intracavitary electrocatheter and an external skin electrode.

3- Determine the impedance at each frequency and fit the values to a custom mathematical model.

4- The fitted parameters are the inputs of an algorithm that outputs a numerical value directly related to the tissue structural integrity. This algorithm may take into account:

- The values and the absolute or relative changes in impedance or admittance magnitude or phase angle (or alternative representations as real and imaginary part of impedance or admittance, or as intrinsic parameters, resistivity, conductivity and permittivity) or in the impedance or admittance model parameters in selected points of the cardiac cycle. In a preferred case, in the systolic and diastolic points determined by synchronism with other physiological signals (ECG, arterial or left ventricular pressure), or in the maximum and minimum of impedance or admittance related signals.
- The shape (slope in selected points, number and type of local maxima and minima, spectral content) of impedance magnitude or phase angle, or their alternative representations as real and imaginary parts, or in the signals corresponding to the time evolution of the impedance model parameters.
- The ratios, differences, areas determined by the aforementioned signals at different frequencies or model parameters or the combination between them and other physiological signals (ECG, arterial or left ventricular pressure).

## Apparatus and method description

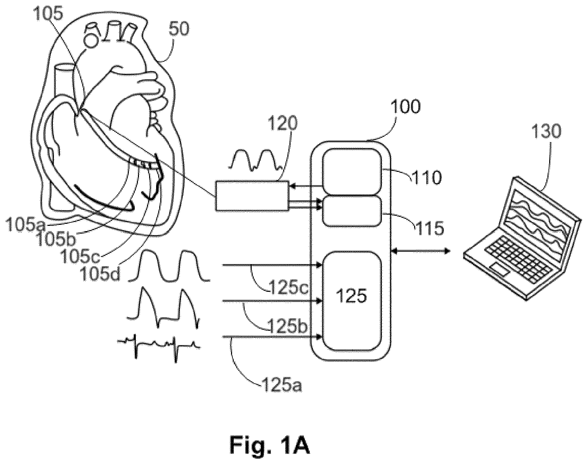

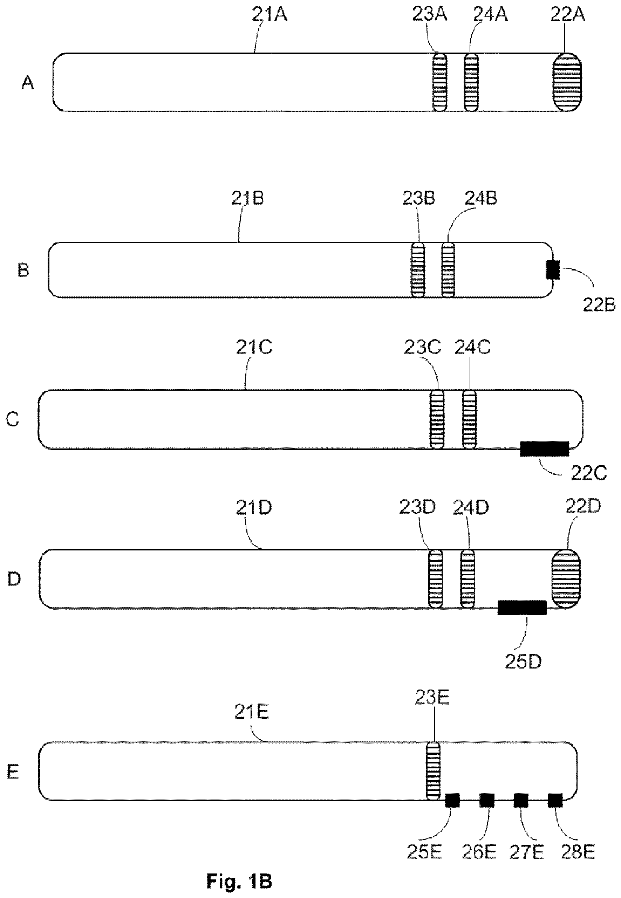

FIG. 1 is a schematic representation of the system used to record simultaneously the phasic changes of myocardial electrical impedance, left ventricular (LV) pressure and surface ECG. Electrical impedance at a myocardium 50 was measured, using a four electrode electrocatheter 105, by applying an alternating current between the outer pair of electrodes (105a, 105d), while the inner two electrodes (105b, 105c) were employed to measure the resulting potential difference. AFE: analog front-end.

The apparatus is made up the following blocks (FIG 1):

1. A main block that comprises an arbitrary waveform generator 110 (AWG-Signal generator) that generates the waveform of the signal to be injected to the myocardium and a digitizer 115 that acquires the signals coming from the front-end (AFE). It also takes care of the synchronism between generation and acquisition. The main block includes a processor to control the sequence of operations.

2. A front-end block 120 that adapts the signal from the AWG-Signal generator 110 to a voltage or current range that fits the electrical safety standards and minimizes the effect of the electrode-tissue impedance. It also includes one or several voltage detection channels to amplify the voltage signals in the catheter and skin electrodes also minimizing the effect of the electrode-tissue impedance. It can also detect and amplify differences between these voltages. A channel to measure the injected current can also be included. The front-end can be adapted to several electrode configurations (2, 3 and 4 electrode configurations) and even switch between them. In cases where more than four electrodes (including skin or electrocatheter electrodes) are used, the frond-end can also select the most appropriate electrodes to inject or detect the signals.

3. A set of electrodes (105a, 105b, 105c, 105d) placed in the tip of a catheter and in the surface of the subject. Preferred electrode combinations can be, for example:

◦ 2 electrodes: one in the catheter tip and one external, in the subject surface
◦ 2 electrodes: one in the catheter tip and one annular near the catheter tip
◦ 3 electrodes: one in the catheter tip, one annular near the catheter tip and one external, in the subject surface.
◦ 4 electrodes: one in the catheter tip, one annular near the catheter tip and two external, in the subject surface.
◦ 2, 3 or 4 electrode technique using two different catheters in different locations of the heart.

4. Additional channels or connection to additional measurement systems if integrated in a higher level apparatus, to acquire ECG, pressure and/or flux signals synchronously with the impedance acquisitions

5. A computer or external processor 130 connected to the apparatus 100 controller through a communication link, running an application that determines the suitable waveform to be uploaded in the AWG 110, the acquisition strategy, that applies the algorithms to obtain the values of the tissue state estimators from the time-frequency characteristics of the measured impedance signals and creates and displays a map of tissue properties that could be merged with the voltage mapping.

FIG. 1B schematically illustrates various electrocatheter configurations A-E that may be used alternatively. Electrocatheter A comprises a body 21A, an electrode 22A and ring electrodes 23A and 24A. Electrode 22A may be used for measuring impedance and/or for ablation purposes. The ring electrodes may be used to detect voltage or to inject current. Electrocatheter B may comprise a body 21B, an electrode 22B and ring electrodes 23B and 24B. Electrode 22A may be smaller than the one used in electrocatheter A and may be used for measuring voltage or for injecting current. The ring electrodes may be used, as previously, to detect voltage or to inject current. Electrocatheter C may be similar to electrocatheter B. It may comprise a body 21C, an electrode 22C and ring electrodes 23C and 24C. However, electrode 22C may be to a lateral position and not at the tip of body 21C and may also be used for measuring impedance or for injecting current. The ring electrodes may be used, as previously, to detect impedance or to inject current. Electrocatheter D may comprise a body 21D, an electrode 22D at the tip of the body 21D, ring electrodes 23D and 24D and a lateral electrode 25D. Electrode 22D may be used for ablation whereas electrode 25D may be used for measuring voltage or for injecting current. The ring electrodes may be used, as previously, to detect voltage or to inject current. Electrocatheter E may comprise a body 21E, one or more ring electrodes 23E and an array of four lateral electrodes 25E, 26E, 27E and 28E. The lateral electrodes may be used for injection or voltage detection and measurement whereas the ring electrodes may be used, as previously, to detect voltage or to inject current, accordingly. Alternatively, the array of four lateral electrodes may comprise a mechanism to separate them from the catheter surface so that they may contact the myocardium tissue without the catheter body coming in contact with the tissue. In another alternative embodiment, the array of four electrodes may be arranged around the tip of the catheter. Another alternative is to use a catheter with many electrodes, for example over an almost cylindrical surface that may be inflated against the surface of a cardiac chamber and to take measurements between selectable electrodes.

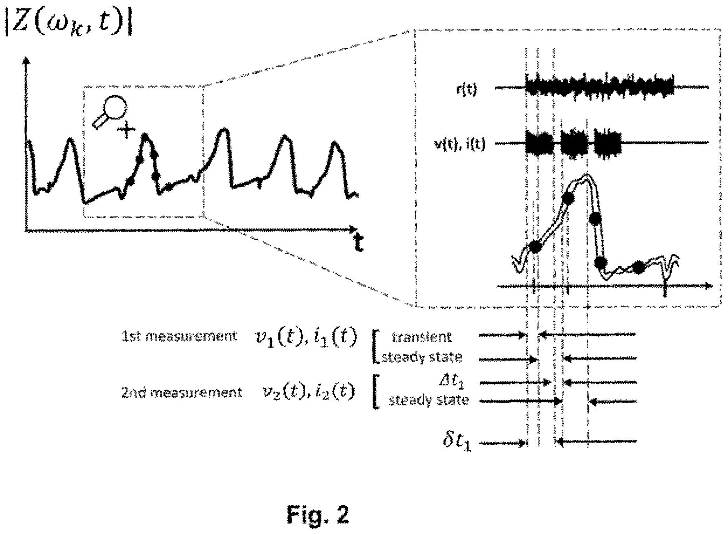

Instead of using a sequence of sinusoidal signals, whose frequency is swept then acquiring information about the impedance at different frequencies in different parts of the cardiac cycle, the fast broadband EIS methods injects bursts or a continuous periodic signal that contains a set of measurement frequencies simultaneously. The minimum length of this signal is one period of the slowest frequency. That is, in a typical case, 1 ms for a minimum frequency of 1 kHz. This would allow acquiring a maximum of 1000 whole impedance spectra per second. Nevertheless, this is usually not needed and a few tenths of spectra per second are enough. The most important is, however, the fact that the acquisition of the resulting voltage and current signals only takes 1 ms, then acquiring a quasi-static picture of the tissue state in a given point of the cardiac cycle. FIG. 2 describes this acquisition method. The voltage and current bursts are drawn longer that they can be for clarity. They can be as short as $1/1000^{th}$ of the approximate cycle period.

FIG. 2 is a representation of the (non-)periodic broadband EIS for the nonparametric-in-time measurement of the time-varying bioimpedance $Z(\omega k,t)$, with $\omega k$ being the excitation (angular) frequency. Two possible approaches exist, where the bio-system is excited either (A) with a continuous reference broadband signal r(t)or (B) with a non-continuous one. (see "Sanchez B et al. A new measuring and identification approach for time-varying bioimpedance using multisine electrical impedance spectroscopy. Physiol Meas. 2013;34:339-57").

There is a variety of signals that allow acquiring a whole spectrum in a given bandwidth: filtered noise, pseudo-random pulse sequences, Discrete Interval Binary Sequences, chirp signals and multisine or multitone signals. This last type of signals, which consist in the addition of a given number of sinusoidal signals, is preferred for our usage because it applies the minimum amount of energy to the tissue given that they only have energy in the selected frequency samples. In other words, for a given energy limit of the injected signal, due to electrical safety reasons, the components of the multisine can have more amplitude than other signals, then allowing to reach a higher signal to noise ratio and then a higher accuracy in the spectrum estimation at the selected frequencies.

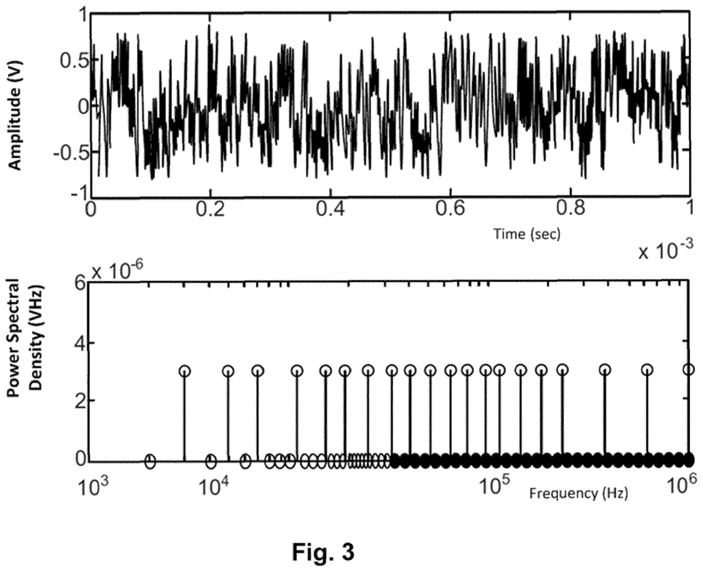

FIG. 3 displays an example of multisine signal and its spectrum. The amplitudes and frequencies can be distributed to reach an optimal adaptation to the expected impedance spectrum and there are several methods to distribute the phase of the multisine components in order to reach a minimum amplitude, for a given RMS signal value given by the amount of frequency components and their corresponding amplitudes. This is usually referred as obtaining a minimal Crest Factor (the ratio between the peak amplitude and the RMS value. Crest factors in the range of 1.5 to 3 can be obtained for multisines with 20-25 components.

With the described apparatus, method and signal, we are able of obtaining any time-frequency impedance feature in the 1 kHz - 1 MHz range and for the times involved in the dynamic behavior of the beating heart.

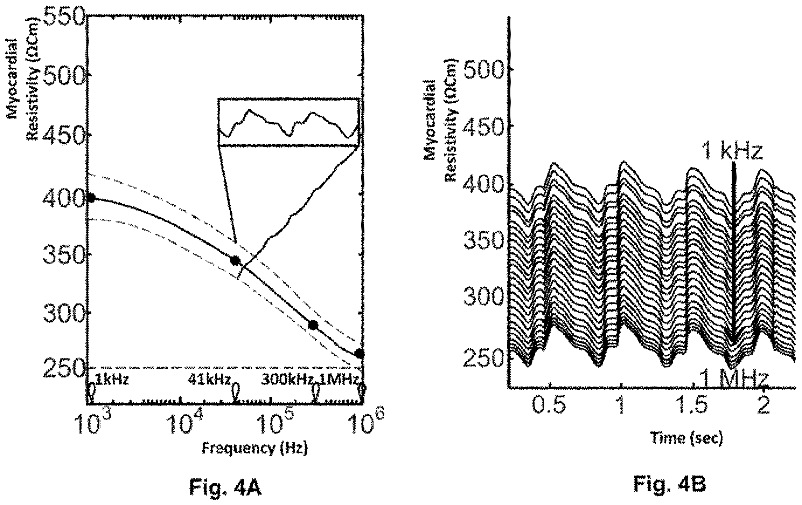

FIG. 4A summarizes this by representing a resistivity spectrum which has a modulation due to myocardium movement, but being now this modulation a source of information, given that the waveform of the impedance as a function of time can be determined at every measured frequency (41 kHz for example in the figure).

FIG. 4B represents the modulation of the resistivity waveform due to myocardium movement. It shows the time course of the impedance as a function of time, represented for all 26 measured frequencies. The mean value clearly changes, but also the signal shapes may change from low frequencies to high frequencies, and may change in a different way if there is a pathology.

If the signals are acquired with enough quality and there is a suitable calibration method to correct the errors induced by electrodes, cables and the acquisition system frequency response, the acquired spectra can be fitted to one or several curves that follow the Cole model and then be parametrized by four parameters. The equation for the impedance representation is the following one:

$$Z(f) = R_\infty + \frac{R_0 - R_\infty}{1 + \left(j\frac{f}{f_c}\right)^\alpha} \qquad \textbf{Equation 1}. \text{ Cole model}$$

The parameter $R_0$ provides information on the extracellular space while $R_\infty$ depends on the total volume and the ratio between them on the cell density. The central relaxation frequency $f_c$ depends on the average cell size and the parameter $\alpha$ is related to the cell shape and size homogeneity. That means that structural information about the tissue can be obtained from the spectra obtained at every cycle point. If represented in the Wessel plane, the impedance spectra of impedance relaxations of biological materials describe circumference arcs.

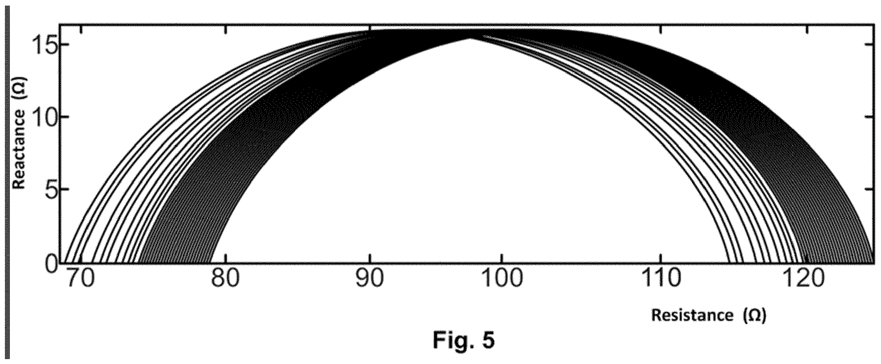

FIG. 5 (Wessel plane) displays the set of arcs corresponding to several cardiac cycles.

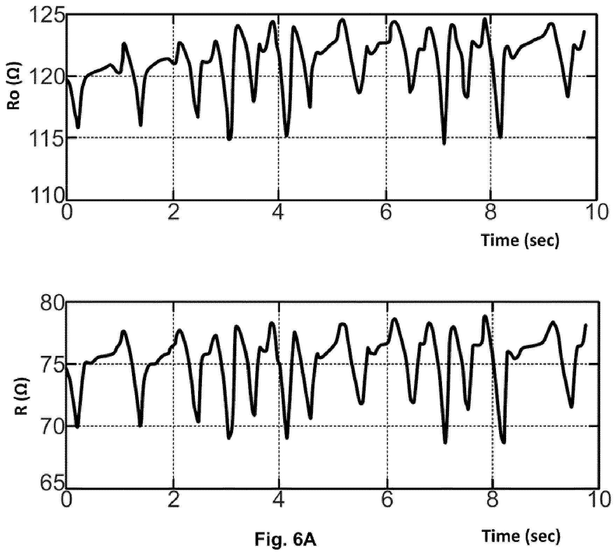

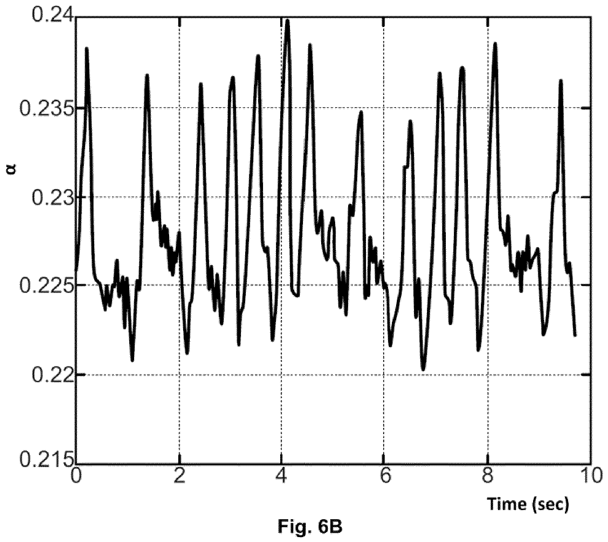

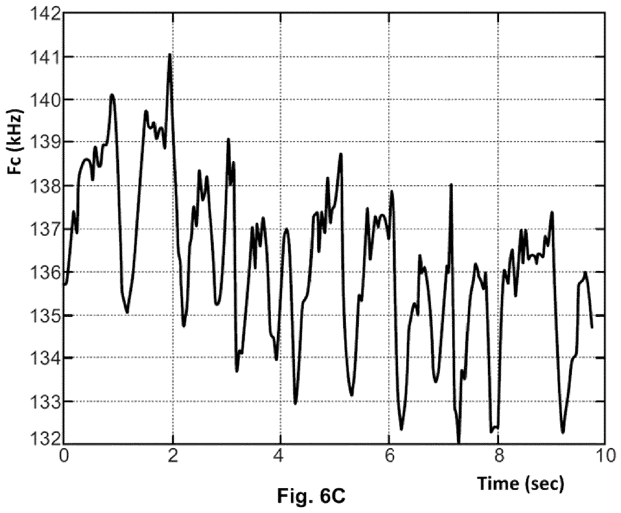

FIG. 6A represents the time course of $R_0$, $R_\infty$, **FIG. 6B** the time course of $\alpha$ and FIG. 6C the time course of $f_c$ obtained by acquiring 60 spectra per second and fitting them to the Cole model for impedance.

Summarizing, with the presented combination of apparatus, acquisition method and signal, it is possible to place a catheter in a given point of the myocardium and, in the time corresponding to a beat cycle, acquire an amount of impedance spectra able to characterize the tissue including time and frequency information, then allowing a better characterization that would help identifying the tissue structural integrity in endocardial or epicardial mapping procedures. This should improve the detection of the areas with non-transmural infarction, which could be arrhythmia foci, in the catheter ablation treatment of malignant ventricular arrhythmias in patients with myocardial infarction.

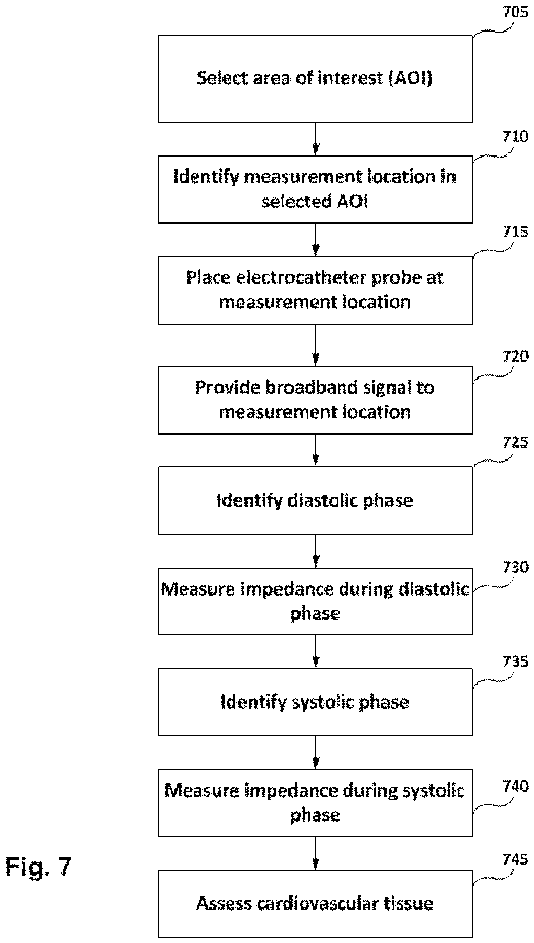

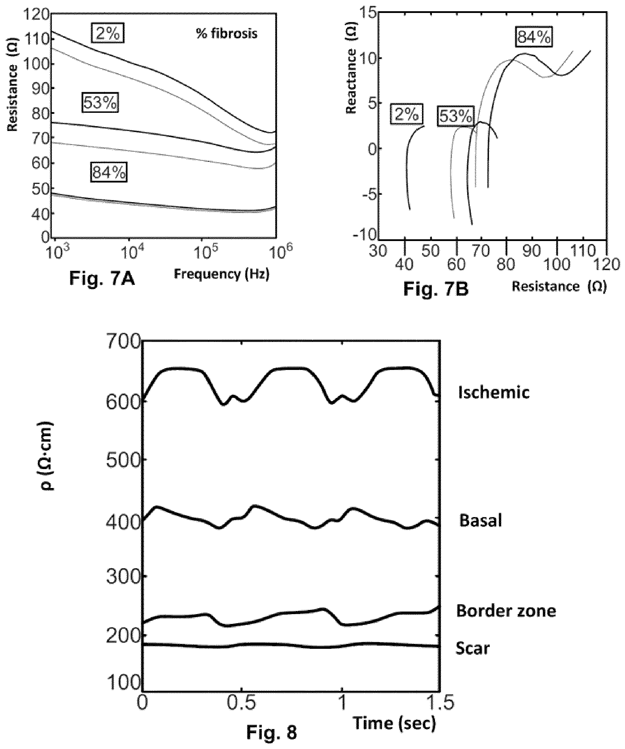

FIG. 7 is a flow diagram of a method of assessing a cardiac tissue. In a first block 705, an area of interest of the cardiac tissue is selected. Then, in block 710, one or more measurement locations in the selected area of interest are identified. In block 715, an electrocatheter probe at the one or more measurement locations is placed. In block 720, a broadband spectrum signal is provided to the one or more measurement locations using the electrocatheter probe. In block 725, a diastolic phase of the cardiac cycle is identified. In block 730 the impedance of the cardiac tissue is measured during the identified diastolic phase. In block 735, a systolic phase of the cardiac cycle is identified. In block 740 impedance of the cardiac tissue is measured during the identified systolic phase. Finally, in block 745, the cardiac tissue is assessed and displayed based on the diastolic and systolic phase impedance measurements.

In the following figures, several examples of signals that can be used to derive estimators of the myocardial state are shown.

**FIG.7A** displays impedance spectra of three tissue states and their modulation range due to myocardium movement in three tissue states, characterized by their fibrosis percentage: normal tissue (2% fibrosis), healed scar (84% fibrosis) and border zone (53% fibrosis).

FIG. 7B shows the arcs in the Wessel plane for the three regions. It can be seen how normal tissue has higher impedance while healed scar has not only lower average impedance but also lower modulation and an almost flat spectrum, revealing its resistive behaviour due to the absence of viable cells. Border zone is similar to the non-transmural infarction areas that can be foci of arrhythmia.

FIG. 8 shows the time-domain of impedance magnitude at a selected frequency (1 kHz) of 4 different tissues states. In this case, also an acute ischemic area is included. It can be seen how the different tissue-state areas provide not only different average impedance values, but also different modulations and different signal shapes

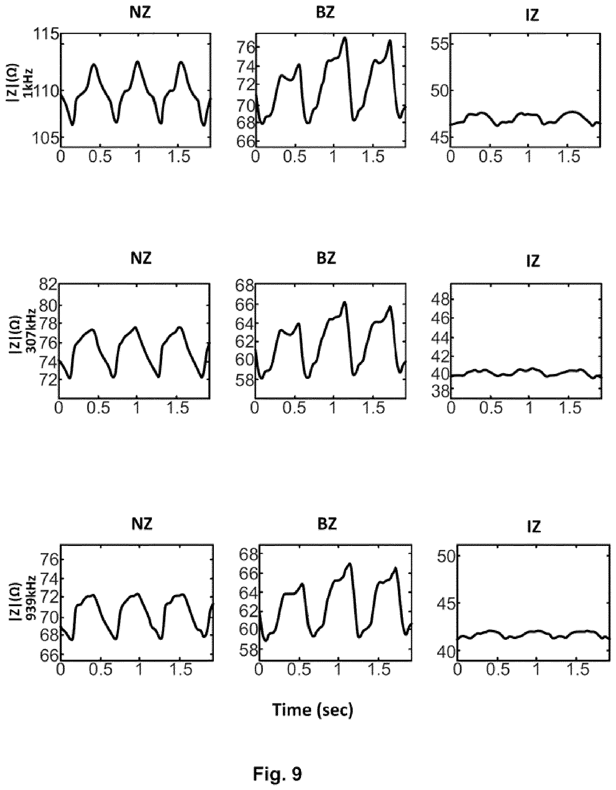

FIG. 9 shows magnitude of the normal (NZ), border (BZ) and healed-scar (infarcted, IZ) zones impedance time signal at three different frequencies (1 kHz, 307 kHz and 939 kHz).

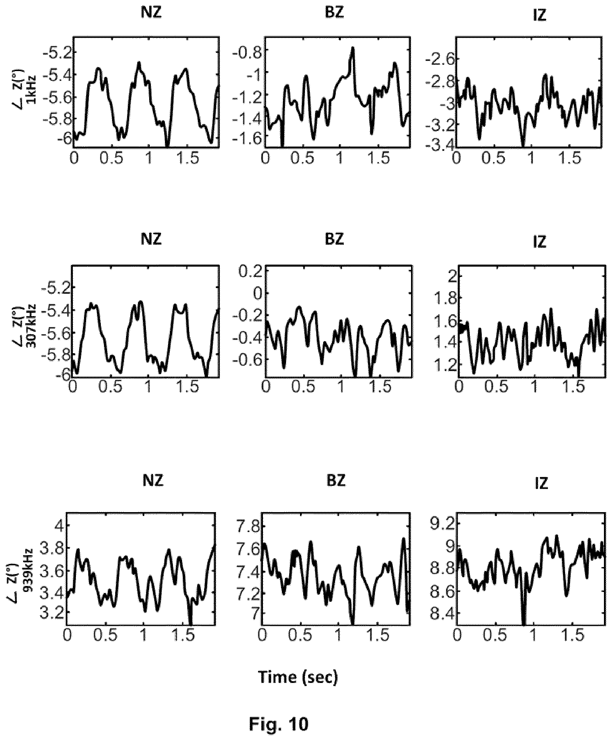

FIG. 10 shows the corresponding phase angle at the same frequencies as the ones indicated in FIG. 9. It can be seen how not only the average value, modulation and signal shape (slope, symmetry, number of local maxima and minima, complexity (spectral content), but also how these features change with frequency. Therefore it is possible to define estimators with more information content and more ability to separate measures using the combined time and frequency content.

Up to the extent of what has been described, the variables involved in the definition of estimators of the tissue state can be not only impedance but also admittance or the related intrinsic parameters, resistivity, conductivity and permittivity. For all of them, the estimators can take into account the values and the absolute or relative changes of the magnitude or phase angle or their alternative representations as real and imaginary part. It can also take into account the model parameters after fitting any of the aforementioned variables spectrum to a mathematical model. Their time course or their values in selected points of the cardiac cycle. In a preferred case, in the systolic and diastolic points determined by synchronism with other physiological signals (ECG, arterial or left ventricular pressure), or in the maximum and minimum of impedance or admittance related signals. Information can also be contained in the delay respect these signals.

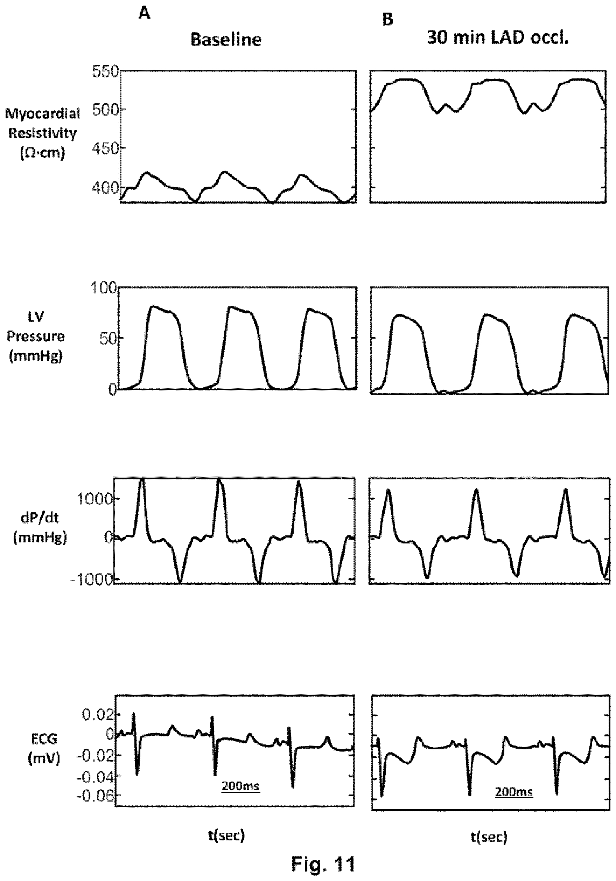

FIG.11 represents the resistivity time course at a frequency of 1 kHz, for two tissue state (normal and acute ischemic) together with the synchronously acquired ventricle pressure, its derivative and the ECG.

Additionally to the possible definition of pathology-dependent delays between the reference signals (ECG, arterial or left ventricular pressure) and the impedance signals, there is a specific representation that gives information about the work performed by the myocardium section that is being measured, and that, consequently, will change depending on the tissue state.

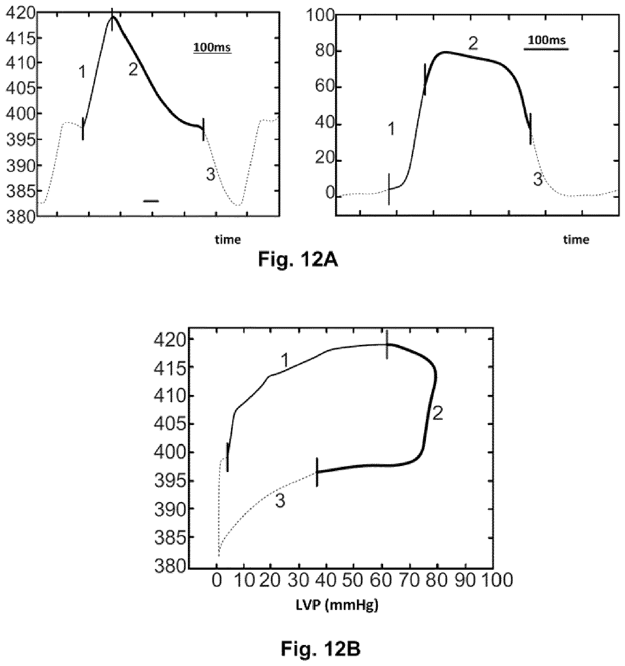

This is represented in FIG. 12A and FIG. 12B. FIG. 12A represents the resistivity at a given frequency (top) and the left ventricular pressure (LVP) (bottom). In FIG. 12B, the cycle described by the evolution of the signal in the Resistivity-LVP plane is shown. This should not be mistaken with the well-known Pressure-Volume diagram, whose area represents the work performed by the whole ventricle. In this case we are representing for the first time a figure whose cycle follows the evolution of a global ventricle variable (the pressure) and the impedance in a localized point. Being the impedance dependent on the myocardium movement in that point, the area of the figure would depend on the work performed by that part of the myocardium. Then it would allow distinguishing active regions (those where the cells contraction provokes the pressure change and where the cells contraction precedes the pressure rise from passive zones, those where tissue deformation is consequence of the pressure change and where both changes are in-phase.

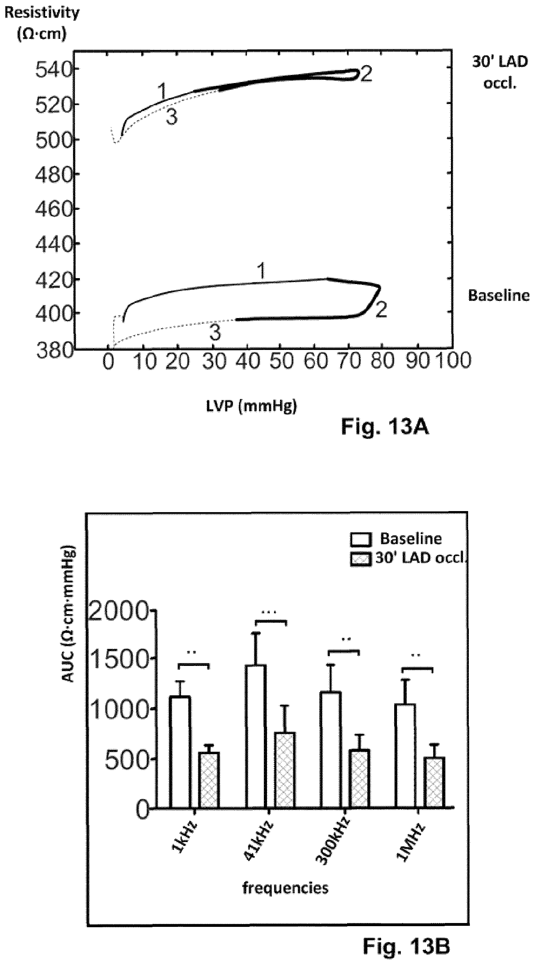

Figures 13A and 13B respectively show the Resistivity-LVP loop at baseline and after 30 min. of left anterior descending (LAD) coronary artery occlusion, and the Resistivity-LVP loop area mean at different frequencies at baseline and after 30 minutes of ischemia. The figures show how the area of the Resistivity-LVP loop is reduced after 30 minutes of acute ischemia compared to its baseline value. Both tissues have mobility, but the ischemic one is following passively the movement induced by the pressure, showing the impedance less delay respect to the pressure and being the Resistivity -LVP diagram near to a line, then having less area. FIG. 13B shows how the separation between both tissue states is better at a given frequency, then having again the time-frequency impedance acquisition an added value.

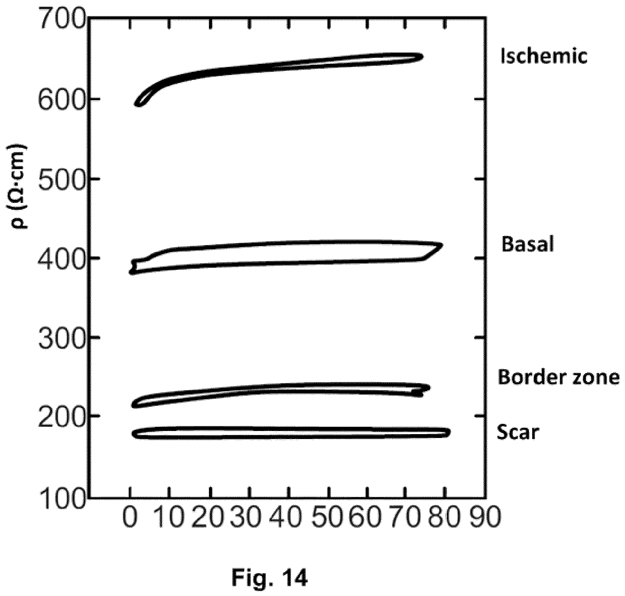

FIG. 14 shows the Resistivity-LVP loop for four different tissue-states. The healed scar not only has lower resistivity but also shows almost no area because it follows the movement passively, then in-phase with the LV pressure. The border-zone, our detection target, has a smaller impedance and impedance modulation that the normal (basal) tissue, but, still having active myocytes, it performs work and shows higher area value.

The obvious fact that the impedance changes are related not only with the tissue state but with the motility can be corroborated by applying drugs that affect motility without destroying the cells.

Myocardial healthy tissue, non-transmural infarct zones and transmural infarct zones can be recognized *in vivo* by specific changes of their myocardial electrical impedance. Myocardial impedance mapping can identify the degree of fibrosis, and therefore the extent and transmurality of the infarct scar. This technique may improve the yielding of catheter ablation of ventricular arrhythmias in patients with chronic myocardial infarction.

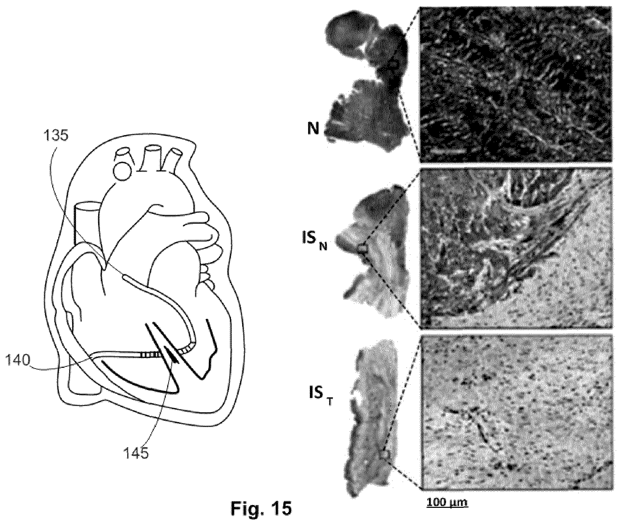

FIG. 15 illustrates a schematic representation of a heart with two electrocatheters; one in the left ventricle 135 and one in the right ventricle 140. The grey area in between the catheters 145 represents an infarct scar in the mid-cardiac septum that could not be detected using the measure of voltage but that can be detected by the impedance spectroscopy measurements.

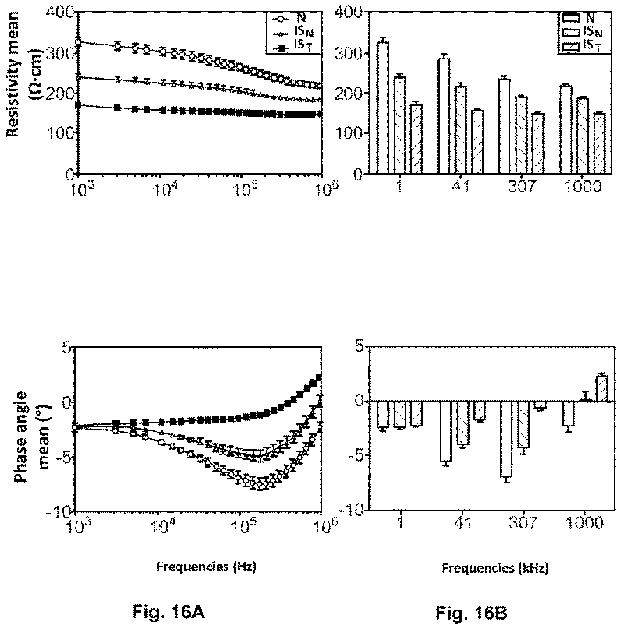

**FIG. 16A** illustrates the mean value of resistivity (top) and phase angle (bottom) vs. the excitation frequency for three different tissues: healthy tissue (N), non-transmural and transmural infarcted scar ($IS_N$ and $IS_T$, respectively). **FIG. 16B** illustrates the mean values of resistivity (top) and phase angle (bottom) at 4 selected frequencies (1, 41, 307 and 1000 kHz) for the three tissues (*** = p<0,001 ; ** = p<0,01).

As shown in **FIG. 16A and 16B,** the mean impedance values during the cardiac cycle of the normal myocardium declines significantly at increasing current frequencies (N: $285.5\pm10.4$ vs. $IS_N$: $216.4\pm7.3$ vs. $IS_T$: $155.5\pm6$ $\Omega \cdot$cm, at 41 kHz; p<0.001) and the phase angle shows a negative relaxation that reaches a maximum at about 307 kHz (N: $-5.6\pm0.4$ vs. $IS_N$: $-4\pm0.4$ vs. $IS_T$: $-1.7\pm0.2°$, at 41 kHz; p<0.001). In contrast, the infarct scar shows lower resistivity values and less marked phase angle deviation at all frequencies.

Moreover, the curves of frequency dependence of resistivity and phase angle depict a progressive attenuation towards the sites as the recording site moves to areas with transmural necrosis. The current frequencies that better differentiate the transmural and non-transmural scar are 1 kHz, 41 kHz and 307 kHz for resistivity and 41 kHz, 307 kHz and 1 MHz for the phase angle.

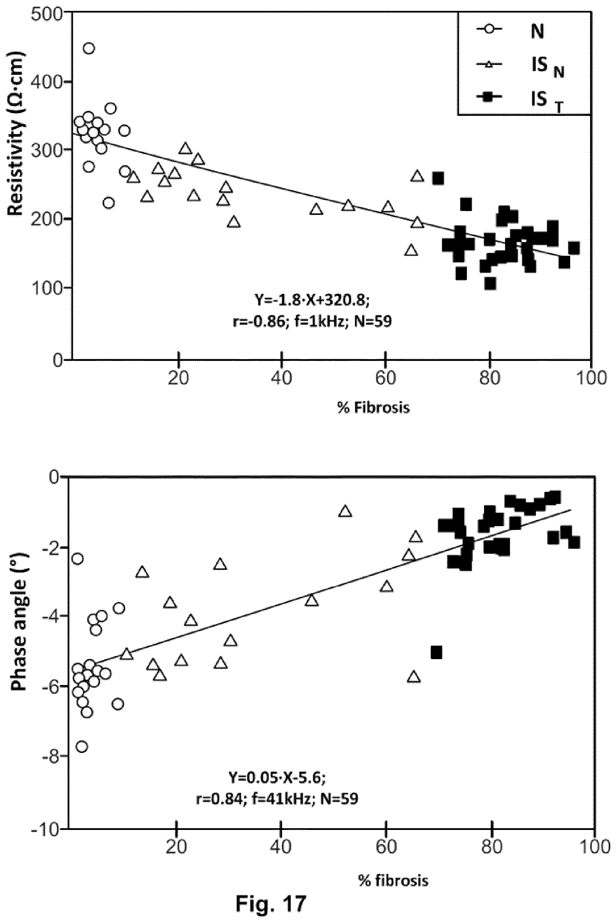

FIG. 17 illustrates a linear correlation between myocardial resistivity (upper-left) / phase angle (lower-left) and the percentage of fibrosis. White filled circles correspond to samples with <10% fibrosis, gray triangles to samples with 10% to 70% of fibrosis and black squares to samples with fibrosis higher than 70%. As illustrated in the figure, and according to the invention, the scope of which is solely defined by the appended claims, tissue samples analyzed taken at the sites with corresponding impedance measurements show that myocardial resistivity correlates negatively with the degree of fibrosis (r=-0.86 at f=1kHz, p<0.001) whereas a positive correlation is observed between phase angle and the fibrotic content (r=0.84 at f=41kHz, p<0.001). The best correlation coefficient is obtained at 1 kHz for myocardial resistivity and 41 kHz for phase angle (Table 1).

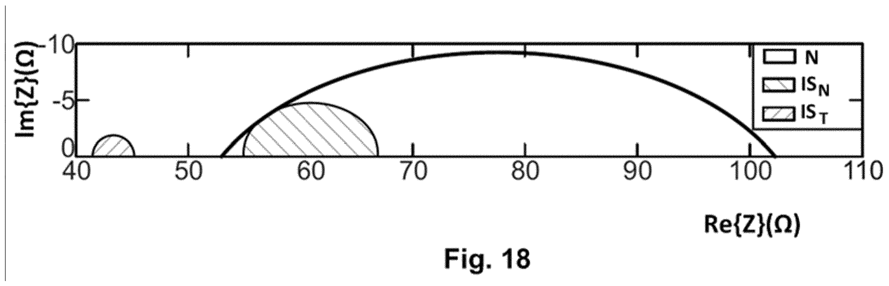

FIG. 18 illustrates the comparative pattern of the Cole impedance model obtained in normal (N: white), non-transmural scar ($IS_N$: grey) and infarcted transmural scar ($IS_T$: black). As the recording site moved from the normal region towards the non-transmural and transmural infarcted area, the Cole arc showed two main changes: a progressive decrease of the area under the curve and a left and down shift towards lower resistivity values (real and imaginary components, respectively). Table 2 summarizes the mean values of the Cole parameters ($R_0$, $R_{inf}$, $\alpha$ and $f_c$) in 59 tissue samples obtained from the 3 index myocardial regions.

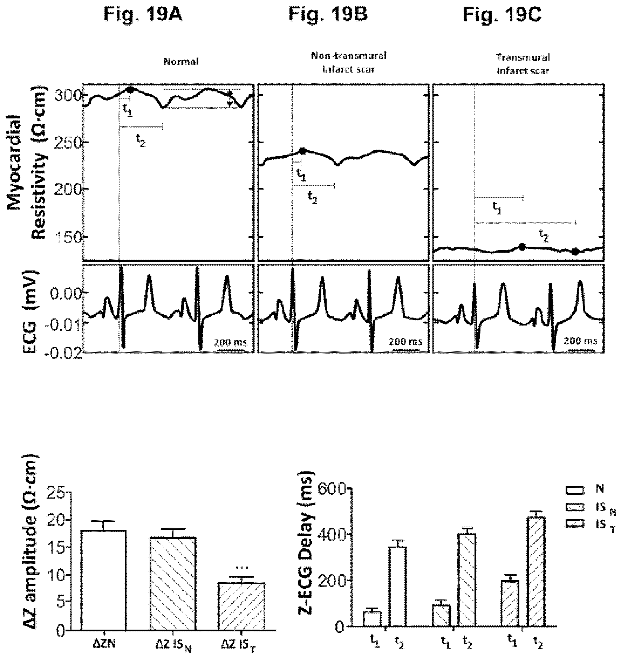

As illustrated in **FIG. 19A,** myocardial tissue electrical resistivity in the normal myocardium depicts a phasic pattern during the cardiac cycle: a peak shortly after the R wave and a minimum value appear in the region of the T wave. As illustrated in **FIG. 19B,** the non-transmural infarcted region depicts a pattern similar to normal myocardium. By contrast, as illustrated in **FIG. 19C** the transmural necrotic region shows a marked attenuation of the magnitude of the resistivity changes with a delayed occurrence of the maximal and minimal resistivity values.

Univariate analysis showed that the impedance parameters that detect differences between the three tissue categories are the mean value of the resistivity and the phase angle during the cardiac cycle at 41 kHz. Using multinomial logistic regression it has been found that the resistivity has the highest probability to correctly classify between healthy tissue, non-transmural and transmural infarct scar.

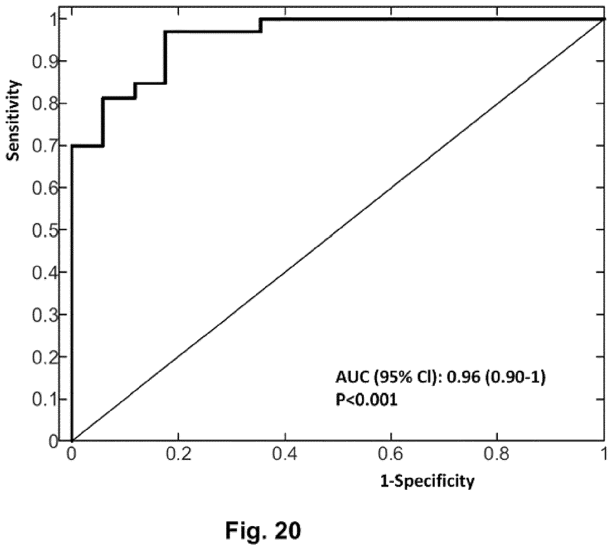

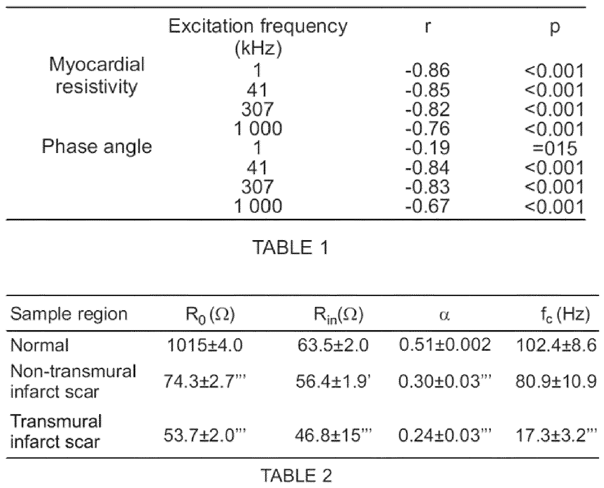

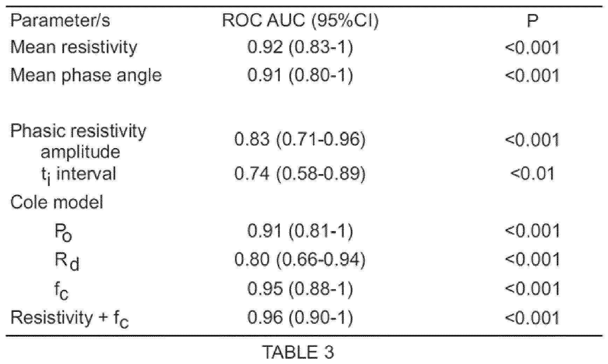

To further assess the predictive ability of different impedance parameters to discriminate between scar tissues with transmural and non-transmural affectation, the analysis of ROC (receiver operating characteristic) curves were used. Table 3 shows the accuracy of the ROC curve assessed by the area under the ROC curve (AUC) for all the different studied variables with its statistical significance levels. Resistivity, phase angle, resistivity amplitude, delay $t_1$, $R_0$, $R_{inf}$, and $f_c$ exhibit good values of AUC with significant p-values, being $f_c$ the best parameter. As illustrated in FIG. 20 which is a multiparametric analysis showing that a combination of resistivity and $f_c$ improve the predictive ability to discriminate between both fibrotic tissues.

Although only a number of examples have been disclosed herein, other alternatives, modifications, uses and/or equivalents thereof are possible. Thus, the scope of the present invention is solely defined by the claims that follow.

**Claims**

1.  A device, comprising:

    means for selecting an area of interest of a cardiac tissue;
    means for identifying measurement locations in the selected area of interest;
    means for placing an electrocatheter probe at the measurement locations;
    means for providing a broadband signal to the measurement locations using the electrocatheter probe;
    means for identifying a diastolic phase of the cardiac cycle;
    means for measuring impedance of the cardiac tissue at the measurement locations during the identified diastolic phase;
means for identifying a systolic phase of the cardiac cycle;
means for measuring impedance of the cardiac tissue at the measurement locations during the identified systolic phase;
means for assessing said cardiac tissue based on said diastolic and systolic impedance measurements at the measurement locations, said means for assessing the cardiac tissue including means for:
identifying a resistivity and a phase angle of the impedance measurements at the measurement locations, and the means for assessing said cardiac tissue further being **characterized by** comprising means for:

determining a fibrosis degree at the measurement locations based on a negative correlation between resistivity and fibrosis degree and on a positive correlation between phase angle and fibrosis degree, and identifying an infarct scar extent and transmurality depending on the determined fibrosis degree at the measurement locations.

2. Device according to claim 1, wherein the means for placing an electrode probe on the measurement locations comprises means for selecting a temporal sequence of measurements on the measurement locations; means for placing the impedance measurement probe successively at each of the measurement locations.

3. Device according to claim 1 or 2, wherein the identified systolic phase comprises a pre-ejection phase and an ejection phase and the means for measuring impedance during the systolic phase comprises means for measuring impedance during the pre-ejection phase; and means for measuring impedance during the ejection phase.

4. Device according to claim 3, wherein the means for measuring impedance comprise two electrocatheters, one configured to be placed at a location of the cardiac tissue and the other at another location of the cardiac tissue or of the body.

5. Device according to claim 4, wherein the means for measuring impedance comprise an electrocatheter configured to be placed at a location of the cardiac tissue and a selected electrode placed at another body location.

6. Device according to any of claims 1 to 5, wherein the broadband signal comprises multiple current pulses with frequencies between 1 kHz and 1 MHz.

7. Device according to any of claims 1 to 6, wherein the means for measuring impedance are means for in vivo measuring impedance.

8. Device according to claim 7, further comprising means for ablating the fibrotic zone.

9. Device according to any of claims 1 to 8, further comprising means for recording of a number of spectra along the cardiac cycle that allow reconstructing time-domain signals at different and simultaneous frequencies and use indicators of the signals shape to assess the cardiac tissue.

10. Device according to claim 9, wherein the indicators of the signals shape comprise one or more of (i) a slope of the signals in selected points, (ii) number and type of local maxima and minima of the signals, and (iii) a spectral content.

11. Device according to any of claims 7 to 10, wherein means for identifying the systolic and diastolic phases use synchronism with other physiological signals comprising one or more of (i) an ECG signal, (ii) a pressure signal and (iii) a flux signal, (iv) a maximum and minimum of impedance or admittance related signals, to identify said phases.

12. Device according to any of claims 7 to 11, wherein the means for measuring impedance comprises means for measuring one or more of the intrinsic variables of impedance.

13. Device according to claim 12, wherein the means for measuring impedance take into account the values and the absolute or relative changes of the magnitude or phase angle or their alternative representations as real and imaginary part, and/or the model parameters after fitting any of the aforementioned variables spectrum to a mathematical model, their time course or their values in selected points of the cardiac cycle.

14. Device according to any of claims 11 to 13, wherein the means for assessing the cardiac tissue are configured to derive assessment from pathology-dependent delays between the reference signals (arterial or left ventricular pressure, ECG) and the impedance related signals.

15. Device according to any of claims 11 to 13, wherein the means for assessing the cardiac tissue are configured to derive assessment from pathology-dependent shape and/or area of the figure resulting of the representation of an impedance-related variable at one or several frequencies and the ventricle pressure.


**Patentansprüche**

1. Eine Vorrichtung umfassend:

   ein Mittel zur Auswahl von einem Bereich von Interesse eines Herzgewebes;
   ein Mittel zur Identifizierung von Messstellen im ausgewählten Bereich von Interesse;
   ein Mittel zur Anordnung von einer Elektrokathetersonde an den Messstellen;
   ein Mittel zur Bereitstellung von einem breitbandigen Signal an die Messstellen durch den Einsatz der Elektrokathetersonde;
   ein Mittel zur Identifizierung von einer diastolischen Phase des Herzzyklus;
   ein Mittel zur Messung der Impedanz des Herzgewebes an den Messstellen während der identifizierten diastolischen Phase;
   ein Mittel zur Identifizierung von einer systolischen Phase des Herzzyklus;
   ein Mittel zur Messung der Impedanz des Herzgewebes an den Messstellen während der identifizieren systolischen Phase;
   ein Mittel zur Beurteilung des Herzgewebes beruhend auf den diastolischen und systolischen Impedanzmessungen an den Messstellen, wobei das Mittel zur Beurteilung des Herzgewebes Mittel umfasst zum:
   Identifizieren von eines spezifischen Widerstands und eines Phasenwinkels der Impedanzmessungen an den Messstellen, und wobei das Mittel zur Beurteilung des Herzgewebes weiterhin **dadurch gekennzeichnet ist, dass** es Mittel umfasst zum:

   Feststellen von einem Fibrosengrad an den Messstellen beruhend auf einer negativen Korrelation zwischen dem spezifischen Widerstand und dem Fibrosengrad und auf einer positiven Korrelation zwischen dem Phasenwinkel und dem Fibrosengrad, und
   Identifizieren von einem Infarktnarbenumfang und Transmuralität in Abhängigkeit von dem festgestellten Fibrosengrad an den Messstellen.

2. Vorrichtung nach Anspruch 1, wobei das Mittel zur Anordnung von einer Elektrodensonde auf den Messstellen ein Mittel zur Auswahl von einer zeitlichen Reihenfolge von Messungen auf den Messstellen, ein Mittel zur sukzessiven Anordnung der Impedanzmesssonde an jeder Messstelle umfasst.

3. Vorrichtung nach Anspruch 1 oder 2, wobei die identifizierte systolische Phase eine Präejektionsphase und eine Ejektionsphase umfasst und das Mittel zur Messung der Impedanz während der systolischen Phase ein Mittel zur Messung der Impedanz während der Präejektionsphase und ein Mittel zur Messung der Impedanz während der Ejektionsphase umfasst.

4. Vorrichtung nach Anspruch 3, wobei das Mittel zur Messung der Impedanz zwei Elektrokatheter umfasst, wobei der eine zur Anordnung an einer Stelle des Herzgewebes und der andere zur Anordnung an einer anderen Stelle des Herzgewebes oder des Körpers konfiguriert ist.

5. Vorrichtung nach Anspruch 4, wobei das Mittel zur Messung der Impedanz einen Elektrokatheter umfasst, der zur Anordnung an einer Stelle des Herzgewebes konfiguriert ist, und eine ausgewählte Elektrode umfasst, die an einer anderen Stelle des Körpers angeordnet ist.

6. Vorrichtung nach einem der Ansprüche 1 bis 5, wobei das breitbandige Signal vielfache Stromimpulse mit Frequenzen von zwischen 1 kHz und 1 MHz umfasst.

7. Vorrichtung nach einem der Ansprüche 1 bis 6, wobei das Mittel zur Messung der Impedanz ein Mittel zur in-vivo-Messung der Impedanz ist.

8. Vorrichtung nach Anspruch 7 weiterhin umfassend ein Mittel zur Ablation des fibrotischen Bereichs.

9.  Vorrichtung nach einem der Ansprüche 1 bis 8, weiterhin umfassend ein Mittel zur Aufnahme von einer Anzahl an Spektren über den Herzzyklus, welche die Rekonstruktion von Zeitsignalen bei verschiedenen und gleichzeitigen Frequenzen ermöglichen und Indikatoren der Signalenform nutzen, um das Herzgewebe zu beurteilen.

10. Vorrichtung nach Anspruch 9, wobei die Indikatoren der Signalenform einen oder mehrere von den folgenden umfassen: (i) eine Steigung der Signale für ausgewählte Punkte, (ii) die Anzahl und die Art der lokalen Maxima und Minima der Signale, und (iii) einen Spektralgehalt.

11. Vorrichtung nach einem der Ansprüche 7 bis 10, wobei das Mittel zur Identifizierung der systolischen und diastolischen Phasen Synchronismus mit anderen physiologischen Signalen umfassend eines oder mehrere von den folgenden nutzen: (i) ein EKG-Signal, (ii) ein Drucksignal und (iii) ein Fluxsignal, (iv) ein Maximum und ein Minimum der auf die Impedanz oder auf die Admittanz bezogenen Signale, um die Phasen zu identifizieren.

12. Vorrichtung nach einem der Ansprüche 7 bis 11, wobei das Mittel zur Messung der Impedanz ein Mittel zur Messung von einer oder mehreren der Eigenvariablen der Impedanz umfasst.

13. Vorrichtung nach Anspruch 12, wobei das Mittel zur Messung der Impedanz die Werte und die absoluten oder die relativen Änderungen der Größe oder des Phasenwinkels oder der alternativen Darstellungen als Real- und Imaginärteil, und/oder die Modellparameter, nachdem eine von den genannten Variablenspektren an ein mathematisches Modell angepasst worden ist, deren Zeitverlauf oder deren Werte für ausgewählte Punkte des Herzzyklus berücksichtigt.

14. Vorrichtung nach einem der Ansprüche 11 bis 13, wobei das Mittel zur Beurteilung des Herzgewebes konfiguriert ist, um eine Beurteilung aus auf die Pathologie bezogenen Verzögerungen zwischen den Bezugssignalen (arteriellem oder linksventrikulärem Druck, EKG) und auf die Impedanz bezogenen Signalen herzuleiten.

15. Vorrichtung nach einem der Ansprüche 11 bis 13, wobei das Mittel zur Beurteilung des Herzgewebes konfiguriert ist, um eine Beurteilung aus der auf die Pathologie bezogenen Form und/oder Fläche der durch die Darstellung von einer auf die Impedanz bezogenen Variable bei einer oder mehreren Frequenzen und des Ventrikeldrucks erhaltenen Figur herzuleiten.

**Revendications**

1.  Un dispositif comprenant :

    un moyen de sélection d'une région d'intérêt d'un tissu cardiaque ;
    un moyen d'identification d'emplacements de mesure dans la région choisie d'intérêt ;
    un moyen pour situer une sonde d'électrocathéter dans les emplacements de mesure ;
    un moyen pour fournir un signal à large bande aux emplacements de mesure en utilisant la sonde d'électrocathéter ;
    un moyen d'identification d'une phase diastolique du cycle cardiaque ;
    un moyen de mesure de l'impédance du tissu cardiaque dans les emplacements de mesure pendant la phase diastolique identifiée ;
    un moyen d'identification d'une phase systolique du cycle cardiaque ;
    un moyen de mesure de l'impédance du tissu cardiaque dans les emplacements de mesure pendant la phase systolique identifiée ;
    un moyen d'évaluation dudit tissu cardiaque à partir desdites mesures d'impédance diastolique et systolique dans les emplacements de mesure, ledit moyen d'évaluation du tissu cardiaque incluant des moyens pour :
    identifier une résistivité et un angle de phase des mesures d'impédance dans les emplacements de mesure,
    et le moyen d'évaluation dudit tissu cardiaque étant **caractérisé en outre en ce qu'**il comprend des moyens pour :

    déterminer un degré de fibrose dans les emplacements de mesure sur la base d'une corrélation négative entre la résistivité et le degré de fibrose et d'une corrélation positive entre l'angle de phase et le degré de fibrose, et
    identifier une ampleur de cicatrice d'infarctus et de la transmuralité en fonction du degré de fibrose déterminé dans les emplacements de mesure.

**2.** Dispositif selon la revendication 1, dans lequel le moyen pour situer une sonde d'électrode sur les emplacements de mesure comprend un moyen pour choisir une séquence temporelle de mesures sur les emplacements de mesure ; un moyen pour situer la sonde de mesure d'impédance dans chacun des emplacements de mesure de manière successive.

**3.** Dispositif selon la revendication 1 ou 2, dans lequel la phase systolique identifiée comprend une phase de pré-éjection et une phase d'éjection et le moyen de mesure de l'impédance pendant la phase systolique comprend un moyen de mesure de l'impédance pendant la phase de pré-éjection ; et un moyen de mesure de l'impédance pendant la phase d'éjection.

**4.** Dispositif selon la revendication 3, dans lequel le moyen de mesure de l'impédance comprend deux électrocathéters, l'un configuré pour être situé dans un emplacement du tissu cardiaque et l'autre dans un autre emplacement du tissu cardiaque ou du corps.

**5.** Dispositif selon la revendication 4, dans lequel le moyen de mesure de l'impédance comprend un électrocathéter configuré pour être situé dans un emplacement du tissu cardiaque et une électrode choisie située dans un autre emplacement du corps.

**6.** Dispositif selon l'une quelconque des revendications 1 à 5, dans lequel le signal à large bande comprend de multiples impulsions de courant avec des fréquences d'entre 1 kHz et 1 MHz.

**7.** Dispositif selon l'une quelconque des revendications 1 à 6, dans lequel le moyen de mesure de l'impédance est un moyen de mesure in vivo de l'impédance.

**8.** Dispositif selon la revendication 7, comprenant en outre un moyen d'ablation de la région fibrotique.

**9.** Dispositif selon l'une quelconque des revendications 1 à 8, comprenant en outre un moyen pour enregistrer un certain nombre de spectres le long du cycle cardiaque qui permettent de reconstruire des signaux dans le domaine du temps à des fréquences différentes et simultanées et qui utilisent des indicateurs de la forme des signaux pour évaluer le tissu cardiaque.

**10.** Dispositif selon la revendication 9, dans lequel les indicateurs de la forme des signaux comprennent un ou plusieurs des suivants (i) une pente des signaux dans des points choisis, (ii) le nombre et le type des maxima et des minima locaux des signaux, et (iii) un contenu spectral.

**11.** Dispositif selon l'une quelconque des revendications 7 à 10, dans lequel le moyen d'identification des phases systoliques et diastoliques utilise du synchronisme avec d'autres signaux physiologiques comprenant un ou plusieurs des suivants : (i) un signal ECG, (ii) un signal de pression et (iii) un signal de flux, (iv) un maximum et un minimum de signaux liés à l'impédance ou à l'admittance, pour identifier lesdites phases.

**12.** Dispositif selon l'une quelconque des revendications 7 à 11, dans lequel le moyen de mesure de l'impédance comprend un moyen de mesure d'une ou de plusieurs variables intrinsèques de l'impédance.

**13.** Dispositif selon la revendication 12, dans lequel le moyen de mesure de l'impédance prend en compte les valeurs et les variations absolues ou relatives de la magnitude ou de l'angle de phase ou leurs représentations alternatives comme partie réelle et imaginaire, et/ou les paramètres de modèle après l'ajustement d'un quelconque des spectres de variables susmentionnés à un modèle mathématique, leur évolution temporelle ou leurs valeurs dans des points choisis du cycle cardiaque.

**14.** Dispositif selon l'une quelconque des revendications 11 à 13, dans lequel le moyen d'évaluation du tissu cardiaque est configuré pour dériver une évaluation à partir de retards dépendants de la pathologie entre les signaux de référence (pression artérielle ou ventriculaire gauche, ECG) et les signaux liés à l'impédance.

**15.** Dispositif selon l'une quelconque des revendications 11 à 13, dans lequel le moyen d'évaluation du tissu cardiaque est configuré pour dériver une évaluation à partir de la forme dépendante de la pathologie et/ou de la surface de la figure obtenue moyennant la représentation d'une variable liée à l'impédance à une ou à plusieurs fréquences et la pression ventriculaire.

**Fig. 1A**

**Fig. 1B**

Fig. 2

**Fig. 3**

Fig. 4B

Myocardial Resistivity (Ωcm)

Time (sec)

1 kHz

1 MHz

Fig. 4A

Myocardial Resistivity (Ωcm)

Frequency (Hz)

1kHz    41kHz    300kHz 1MHz

Fig. 5

Fig. 6A

Fig. 6B

Fig. 6C

Fig. 7

705 — Select area of interest (AOI)

710 — Identify measurement location in selected AOI

715 — Place electrocatheter probe at measurement location

720 — Provide broadband signal to measurement location

725 — Identify diastolic phase

730 — Measure impedance during diastolic phase

735 — Identify systolic phase

740 — Measure impedance during systolic phase

745 — Assess cardiovascular tissue

**Fig. 7A**

**Fig. 7B**

**Fig. 8**

Fig. 9

Fig. 10

Fig. 11

Fig. 12A

Fig. 12B

**Fig. 13A**

**Fig. 13B**

**Fig. 14**

Fig. 15

Fig. 16A

Fig. 16B

Fig. 17

Fig. 18

# Fig. 19A

# Fig. 19B

# Fig. 19C

AUC (95% CI): 0.96 (0.90-1)
P<0.001

Fig. 20

| | Excitation frequency (kHz) | r | p |
|---|---|---|---|
| Myocardial resistivity | 1 | -0.86 | <0.001 |
| | 41 | -0.85 | <0.001 |
| | 307 | -0.82 | <0.001 |
| | 1 000 | -0.76 | <0.001 |
| Phase angle | 1 | -0.19 | =015 |
| | 41 | -0.84 | <0.001 |
| | 307 | -0.83 | <0.001 |
| | 1 000 | -0.67 | <0.001 |

TABLE 1

| Sample region | $R_0$ ($\Omega$) | $R_{in}$ ($\Omega$) | $\alpha$ | $f_c$ (Hz) |
|---|---|---|---|---|
| Normal | 1015±4.0 | 63.5±2.0 | 0.51±0.002 | 102.4±8.6 |
| Non-transmural infarct scar | 74.3±2.7''' | 56.4±1.9' | 0.30±0.03''' | 80.9±10.9 |
| Transmural infarct scar | 53.7±2.0''' | 46.8±15''' | 0.24±0.03''' | 17.3±3.2''' |

TABLE 2

| Parameter/s | ROC AUC (95%CI) | P |
|---|---|---|
| Mean resistivity | 0.92 (0.83-1) | <0.001 |
| Mean phase angle | 0.91 (0.80-1) | <0.001 |
| Phasic resistivity amplitude | 0.83 (0.71-0.96) | <0.001 |
| $t_i$ interval | 0.74 (0.58-0.89) | <0.01 |
| Cole model | | |
| $P_o$ | 0.91 (0.81-1) | <0.001 |
| $R_d$ | 0.80 (0.66-0.94) | <0.001 |
| $f_c$ | 0.95 (0.88-1) | <0.001 |
| Resistivity + $f_c$ | 0.96 (0.90-1) | <0.001 |

TABLE 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5494042 A
- US 2001018608 A
- US 5485849 A

### Non-patent literature cited in the description

- **WARREN M et al.** Percutaneous electrocatheter technique for on-line detection of healed transmural myocardial infarction. *Pacing Clin Electrophysiol,* 2000, vol. 23, 1283-1287
- **FRANCONE M. et al.** Impact of primary coronary angioplasty delay on myocardial salvage, infarct size, and microvascular damage in patients with ST-segment elevation myocardial infarction. Insight from cardiovascular magnetic resonance. *J. Am. Coll. Cardiol.,* 2009, vol. 54, 2145-2153
- **ARENAL A et al.** Tachycardia-related channel in the scar tissue in patients with sustained monomorphic ventricular tachycardias: Influence of the voltage scar definition. *Circulation,* 2004, vol. 110, 2568-2574
- Characterization of the arrhythmogenic substrate in ischemic and nonischemic cardiomyopathy. Implications for catheter ablation of hemodynamically unstable ventricular tachycardia. **NAKAHARA S. et al.** J. Am. Coll. Cardiol. Elsevier Inc, 2010, vol. 55, 2355-2365
- **YAN A.T. et al.** Characterization of the peri-infarct zone by contrast-enhanced cardiac magnetic resonance imaging is a powerful predictor of post-myocardial infarction mortality. *Circulation,* 2006, vol. 114, 32-39
- **CODREANU A. et al.** Electroanatomic characterization of post-infarct scars. Comparison with 3-Dimensional myocardial scar reconstruction based on magnetic resonance imaging. *J. Am. Coll. Cardiol.,* 2008, vol. 52, 839-842
- **KLEIN C. et al.** *Assessment of myocardial viability with contrast-enhanced magnetic resonance imaging: comparison with positron emission tomography,* 2002, vol. 105, 162-167
- **SPERELAKIS N. et al.** Electrical impedance of cardiac muscle. *Circ. Res.,* 1961, vol. 9, 1280-1283
- **CINCA J. et al.** Passive transmission of ischemic ST segment changes in low electrical resistance myocardial infarct scar in the pig. *Cardiovasc. Res.,* 1998, vol. 40, 103-112
- **FALLERT MA et al.** Myocardial electrical impedance mapping of ischemic sheep hearts and healing aneurysms. *Circulation,* 1993, vol. 87, 199-207
- **SALAZAR Y et al.** Transmural versus nontransmural in situ electrical impedance spectrum for healthy, ischemic, and healed myocardium. *IEEE Trans. Biomed. Eng.,* 2004, vol. 51, 1421-1427
- **SANCHEZ B et al.** A new measuring and identification approach for time-varying bioimpedance using multisine electrical impedance spectroscopy. *Physiol. Meas.,* 2013, vol. 34, 339-57
- **JORGE E. et al.** Early detection of acute transmural myocardial ischemia by the phasic systolic- diastolic changes of local tissue electrical impedance. *Am. J. Physiol. Heart Circ. Physiol.,* 2015, vol. 310, H436-H443
- **SANCHEZ B. et al.** A new measuring and identification approach for time-varying bioimpedance using multisine electrical impedance spectroscopy. *Physiol. Meas.,* 2013, vol. 34, 339-57
- **SANCHEZ B et al.** A new measuring and identification approach for time-varying bioimpedance using multisine electrical impedance spectroscopy. *Physiol Meas.,* 2013, vol. 34, 339-57